# EUROPÄISCHE PATENTSCHRIFT

(11) **EP 2 808 084 B1**
(45) Veröffentlichungstag und Bekanntmachung des Hinweises auf die Patenterteilung: **16.11.2016**
(21) Anmeldenummer: 14001650.2
(22) Anmeldetag: 10.05.2014
(51) Int. Cl.: B01L 7/00, C12M 1/00

(54) **Inkubationskammer**
Incubation chamber
Chambre d'incubation

(30) Priorität: 31.05.2013 DE 102013009136
(43) Veröffentlichungstag der Anmeldung: 03.12.2014
(73) Patentinhaber: SunChrom GmbH, 61381 Friedrichsdorf (DE)
(72) Erfinder: Barka, Günes, 61381 Friedrichsdorf (DE); Bäumlisberger, Dominic, 61440 Oberursel (DE)
(74) Vertreter: Patentanwälte Olbricht Buchhold Keulertz

(56) Entgegenhaltungen:
- EP-A1- 0 238 313
- WO-A2-01/40436
- DE-A1- 2 259 377
- US-A- 5 519 188

## Beschreibung

Die vorliegende Erfindung betrifft eine Inkubationskammer mit einem Feuchtigkeitsreservoir, einer Klimatisierungseinrichtung, die die Einstellung einer gewünschten Temperatur ermöglicht, sowie einer Halterung für biologische oder chemische Proben.

Inkubationskammern, die auch als Inkubationsschränke bezeichnet werden, werden eingesetzt, um Bakterien, andere Mikroorganismen und Ziellinien auf geeigneten Medien zu kultivieren, d. h. in der Regel zu vermehren. Sie können auch dazu dienen, Verdauvorgänge von Proteinen oder größeren Peptiden durch bestimmte proteolytische Enzyme, wie z. B. Trypsin, Chemotrypsin, Elastase usw. in kleinere Peptide oder Teile des Ausgangsmaterials unter reproduzierbaren Bedingungen ablaufen zu lassen. Die kleineren Peptidmoleküle haben gänzlich andere chemische Eigenschaften und sind viel einfacher handhabbar, so dass ihre Struktur leichter bestimmbar ist.

Werden Proteine enzymatisch verdaut, und die daraus resultierenden Peptide durch chemische oder durch andere physikalisch-chemische Methoden analysiert sowie deren Aminosäuresequenzen bestimmt, lassen sich mittels verschiedener Techniken die Proteinprimärsequenzen bestimmen. Man spricht in der Proteomanalyse dann auch von einem sogenannten Bottom-Up Ansatz. Für eine umfassende Analyse der Primärsequenzen von Proteinen stellt diese Methode den Stand der Technik dar.

Die Verdauenzyme sind von ihrer Struktur her selbst Proteine oder proteinähnliche Verbindungen. Wie alle Proteine sind sie sehr empfindlich gegen chemische oder physikalische Änderungen in ihrer Umgebung. Viele verlieren ihre Aktivitäten, wenn sie austrocknen, überhitzt werden oder mit organischen Lösungsmitteln in Berührung kommen. Der Verdau von Proteinen erfolgt in Lösung, in Gelmatrizes, auf Membranen oder im Zusammenhang mit der bildgebenden Massenspektrometrie in Gewebeschnitten. Dabei müssen bestimmte Voraussetzungen erfüllt werden, wozu z. B. die Natur der Lösung bzw. der Lösungsmittel, der pH-Wert, der Salzgehalt, die Temperatur und ggf. weitere Randbedingungen gehören.

Die Struktur- und Sequenzaufklärung von Proteinen ist die primäre Voraussetzung für die Erkennung ihrer Funktion im lebenden Organismus. Dafür ist es erforderlich, nicht nur die Sequenz der Aminosäurebausteine zu kennen. Diese Eigenschaften werden heute durch verschiedene massenspektrometrische Techniken untersucht und aufgeklärt. Neben Peptiden können aber auch die noch intakten Proteine mittels Massenspektrometrie analysiert werden, um das genaue Molekulargewicht zu bestimmen und mögliche Degradierungen zu erkennen.

In den letzten Jahren hat sich die Bildgebende Massenspektrometrie etabliert, die es ermöglicht, Proteine direkt zu erkennen und zu bestimmen/zu detektieren, ohne sie durch Extraktion zuvor aus ihrem originären Umfeld entfernen zu müssen. Dabei werden u.a. unterschiedliche Ionisationsmethoden verwendet, wie MALDI (Matrix-assisted Laser Desorption/Ionization- Matrix-unterstützte Laser-Desorption/Ionisation), DESI (Desorption Elektrospray Ionisation - Desorptions Elektrospray Ionisation), SIMS (Secondary ion mass spectrometry - Sekundärionen-Massenspektrometrie). Diese Methode hat den großen Vorteil, dass man dadurch die örtliche Verteilung der einzelnen Proteine erfassen kann, wobei die örtliche Zuordnung zu bestimmten Bereichen eines Gewebes in z. B. Nieren, Leber oder Gehirn dem Wunsch Rechnung trägt, Näheres über die Funktion der Proteine zu erfahren.

Die Massenanalyse von intakten Proteinen direkt aus Geweben ist im Vergleich zu kleineren Molekülen deutlich schwieriger. Im Gegensatz zu Proteinen lassen sich die Peptide besser aus Geweben extrahieren, in Matrixkristalle einbauen und im Massenspektrometer ionisieren. Um aber die aus einem Protein durch enzymatischen Verdau resultierenden Peptide im originären Ort des Gewebes zu belassen, müssen während des Verdauprozesses bestimmte Kriterien eingehalten werden.

Zunächst ist es wichtig, dass der Verdau in einem bestimmten Temperaturbereich durchgeführt wird, der in der Regel in einem Bereich zwischen 25 und 50°C liegt, weil in diesem Temperaturbereich für die meisten Enzyme die ideale Reaktionstemperatur mit maximalem Reaktionsumsatz und minimalem Aktivitätsverlust liegt. Erforderlich für den Verdauprozess ist Wasser, wobei zu viel Wasser auf oder im Gewebe bewirkt, dass die Peptide ihren originären Platz verlassen und in alle Richtungen diffundieren. Dies führt zu einem Verlust der Ortsauflösung und damit der Möglichkeit getrennte Information von nah bei einander liegenden Arealen auf einem Gewebe zu erhalten, so dass die damit verbundenen wertvollen Informationen verloren gehen.

Um dies sicherzustellen, muss im Gewebe ein konstanter, leicht kondensierender, aber nicht zu hoher, auf der anderen Seite aber auch nicht zu niedriger Wassergehalt vorhanden sein. Idealerweise liegt eine gesättigte Feuchtigkeit nicht nur an der Oberfläche sondern auch in tieferen Bereichen des Gewebes vor, die ausreichend für die enzymatische Reaktion über eine längere Zeit bis zu mehreren Stunden ist.

Es gibt einige Publikationen, die Inkubationskammern vorschlagen, die für den jeweiligen Anwendungsfall in der Art eines Provisoriums geschaffen werden, wobei auch gelegentlich der gänzliche Verzicht auf Inkubatoren durch besondere Techniken der Enzymzugabe vorgeschlagen wird. Bei den bekannten Inkubationskammern ist über die Ausbeute der Verdauprozesse wenig berichtet worden, wobei allerdings direkt oder indirekt Probleme berichtet werden, die das Einhalten der zuvor erörterten Randbedingungen betreffen. Ein großes Problem stellt auch die Wasserkondensation in der Inkubationskammer dar, da herabfallende oder fließende Wassertropfen das Gewebe überfluten können.

Eine Inkubationskammer mit mehreren Heizelementen ist aus der US 5,519,188 bekannt. Weitere Inkubationskammern finden sich in der EP 0 238 313 A1, der WO 01/40436 A2 und der DE 2259377 A1.

Die Aufgabe der vorliegenden Erfindung besteht darin, eine Inkubationskammer zu schaffen, die z.B. den Ablauf der erörterten Verdauprozesse unter definierten und reproduzierbaren Bedingungen gewährleistet.

Erfindungsgemäß wird die Aufgabe durch eine Inkubationskammer nach Anspruch 1gelöst. Die Klimatisierungseinrichtung verfügt über zwei getrennte Klimatisierungszonen, die getrennt voneinander regelbar sind, wobei eine erste Zone im Bereich des Deckels der Inkubationskammer angeordnet ist und in der Inkubationskammer sind Temperatur- und Feuchtigkeitssensoren vorgesehen, die die Heizleistung und/oder die Lüfterleistung in Abhängigkeit von den gemessenen Werten über eine Steuervorrichtung steuern.

Es hat sich gezeigt, dass sich durch die Anordnung einer Klimatisierungszone im Bereich des Deckels die Randbedingungen, unter welchen beispielsweise ein Verdauprozess ablaufen soll, leichter einhalten lassen, wobei die getrennte Temperaturregelung im Bereich des Deckels die dortige Bildung von Tropfen in Folge von Wasserkondensation sicher vermeiden hilft. Vom Deckel auf das Gewebe herabfallende Wassertropfen stellen damit kein Problem mehr dar. Durch das Halten einer optimalen Temperatur werden die Verdauprozesse optimiert und können in minimaler Zeit ein optimales Ergebnis erreichen. Ortsinformationen in Gewebeproben können sicher erhalten werden.

Durch die Sensoren und die Steuervorrichtung lassen sich alle Parameter in Echtzeit beeinflussen, wobei bestimmte Vorgabewerte, beispielsweise die Temperaturdifferenz zwischen der ersten und der zweiten Klimatisierungszone vorab gespeichert sein können.

In einer bevorzugten Ausführungsform der Erfindung ist vorgesehen, dass wenigstens eine der beiden Klimazonen auch eine Kühlfunktion aufweist. Auf diese Weise ist es möglich, die Temperatur in der Kammer beispielsweise bis auf 4°C herunterzukühlen, was sich als Temperatur für die Objekthalterung und die darin gehaltenen Objekt- und Probenträger für den Enzymauftrag als vorteilhaft erweist. Nach dem Erwärmen der Inkubationskammer für den enzymatischen Verdau kann die Kammer dann auch wieder langsam gleichmäßig heruntergekühlt werden, ohne dass es zu einer unerwünschten Wasserkondensation innerhalb der Kammer kommt, die insbesondere auf den Proben, wie z. B. dort angeordneten Gewebeschnitten stattfinden könnte. In einer weiteren bevorzugten Ausführungsform der Erfindung ist vorgesehen, dass die zweite Klimatisierungszone, die vorzugsweise auch die Kühlfunktion ermöglicht, in der Bodenplatte der Inkubationskammer angeordnet ist. Diese zweite Klimatisierungszone kann in ihrer Leistungsfähigkeit auch stärker ausgebildet sein, da sie primär für das Einstellen einer bestimmten Temperatur von z. B. bis zu 60°C in der Inkubationskammer zur Verfügung stehen kann, während die erste Klimatisierungszone im Deckel durch gezielte Temperierung der Innenseite des Deckels hauptsächlich der Bildung von Kondensattropfen an dieser Stelle entgegenwirkt.

Das Feuchtigkeitsreservoir der Inkubationskammer ist vorzugweise als Wanne ausgebildet, deren aktive Oberfläche durch eine Filz- oder Schaumstofflage oder andere Wasser speichernde Stoffe vergrößert ist. Diese Wanne kann wie die Objekthalterung aus wärmeleitfähigem Metall und/oder Kunststoff bestehen, wobei Objekthalter und Wanne auch einstückig ausgebildet sein können. Der Objekthalter ist so gestaltet, dass die Objektträger und/oder Probenträger in einem bestimmten Abstand zum Feuchtigkeitsreservoir gehalten sind. Gegebenenfalls kann die Halterung so beschaffen sein, dass die Objektträger oder Probenträger wahlweise mit nach unten zum Reservoir oder nach oben zum freien Raum hin liegenden Proben anordbar sind.

In einer besonders bevorzugten Ausführungsform der Erfindung ist eine Lüftervorrichtung vorgesehen. Eine Lüftervorrichtung bietet den Vorteil, dass sich durch gezielte Luftumwälzung in allen Bereich der Kammer homogene Temperatur und Feuchtigkeitseigenschaften bereitstellen lassen und die Verdunstungsgeschwindigkeit beeinflusst werden kann. Vorzugsweise ist die die Lüfterleistung dabei zwischen 0 und einem Maximalwert einstellbar, da sie zur Einstellung der Umgebungsbedingungen herangezogen werden kann. So kann bei einer zu hohen gemessenen Feuchte die Lüfterleistung erhöht werden, um die Kondensationsvorgänge im Bereich der aktiven Probenoberfläche reduzieren.

Der Deckel der Inkubationskammer sollte aus Gründen der Zugänglichkeit abnehmbar oder wenigstens aufschwenkbar sein.

Nachfolgend wird anhand der beigefügten Zeichnungen näher auf ein Ausführungsbeispiel der Erfindung eingegangen. Es zeigen:
- Fig. 1: eine geschnittene Darstellung einer Inkubationskammer;
- Fig. 2: einen Schnitt in der Lüfterebene;
- Fig. 3: einen gegenüber Fig. 1 gedrehten Schnitt der Inkubationskammer;

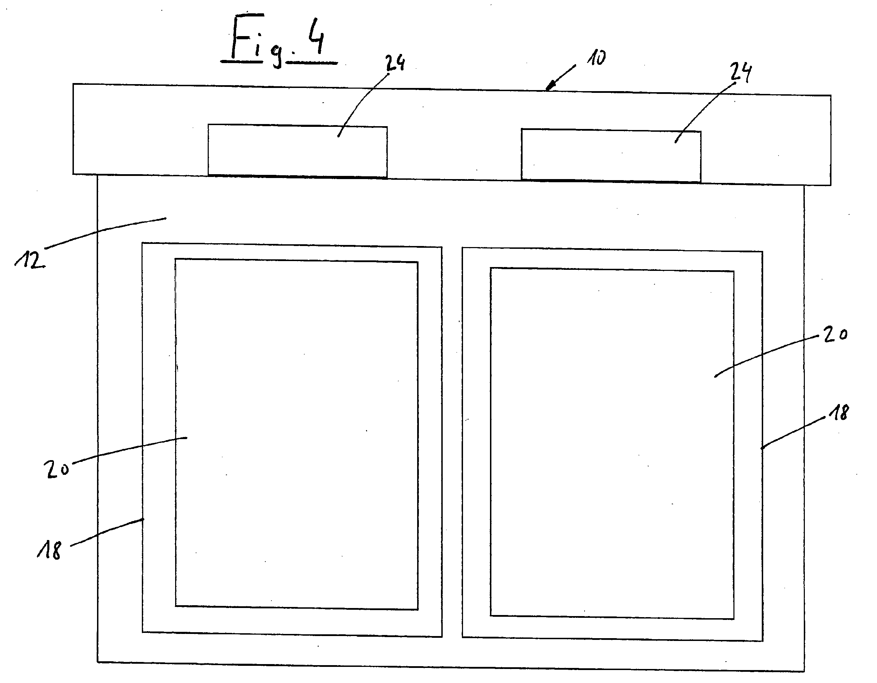

- Fig. 4: eine Schnittdarstellung der Inkubationskammer in der Ebene des Objektträgerhalters.

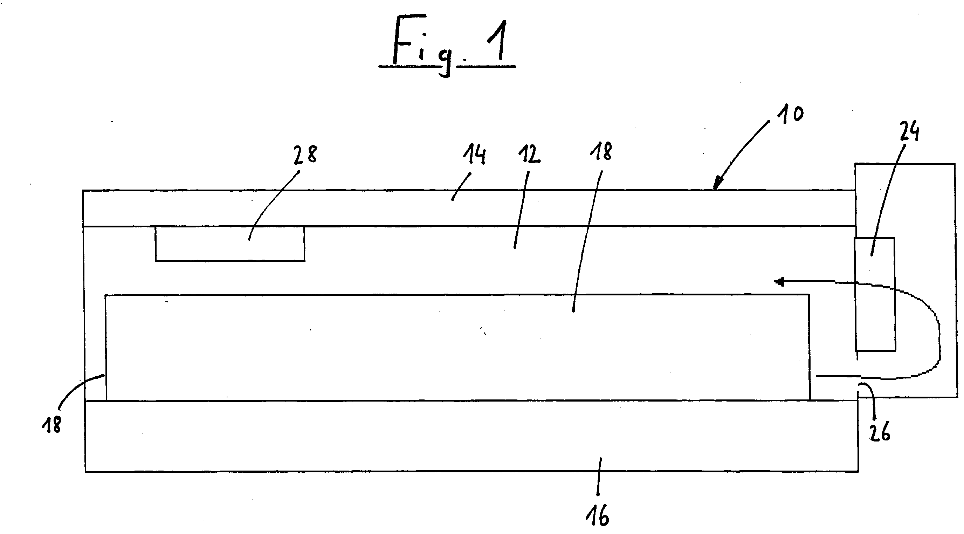

In Fig. 1 ist eine Inkubationskammer 10 gezeigt, die einen allseits umschlossenen Raum 12 aufweist. In einem abnehmbaren Deckel 14, sind zur Ausbildung einer ersten Klimatisierungszone elektrische Heizelemente (nicht im Einzelnen dargestellt) vorgesehen. In einer Bodenplatte 16 sind Peltier-Elemente vorgesehen, die als Kühl- oder Heizelemente zur Ausbildung einer zweiten Klimatisierungszone eingesetzt werden. In der Kammer 12 ist ein Objektträgerhalter 18 angeordnet, auf welchem Objektträger 20 oder MALDI-Probenträger oder sonstige Träger angeordnet werden können.

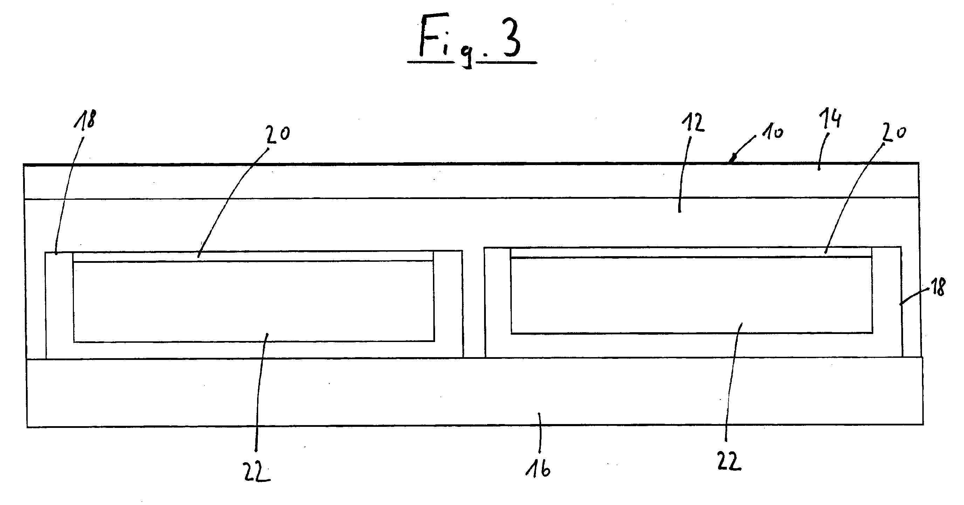

Der Objekthalter 18 ist, wie aus Fig. 3 gut zu erkennen ist, als Wasserreservoir 22 ausgebildet, wobei dieses Wasserreservoir mit destilliertem Wasser oder Wassergemischen gefüllt ist, so dass es das für die Erzeugung der Luftfeuchtigkeit notwendige Feuchtigkeitsreservoir bildet. Zum Vergrößern der wirksamen Oberfläche ist das Wasser wenigstens teilweise in einer Wasser aufnehmenden und speichernden Schicht beispielsweise aus Filz oder Schaumstoff oder sonstigen geeigneten Materialien versehen. Eine entsprechende Lage kann auch auf dem Wasser aufliegen. Zwischen der Filz- oder Schaumstoffschicht und den Objektträgern 20 verbleibt ein Abstand, so dass eine unmittelbare Berührung des Objektträgers ausgeschlossen ist.

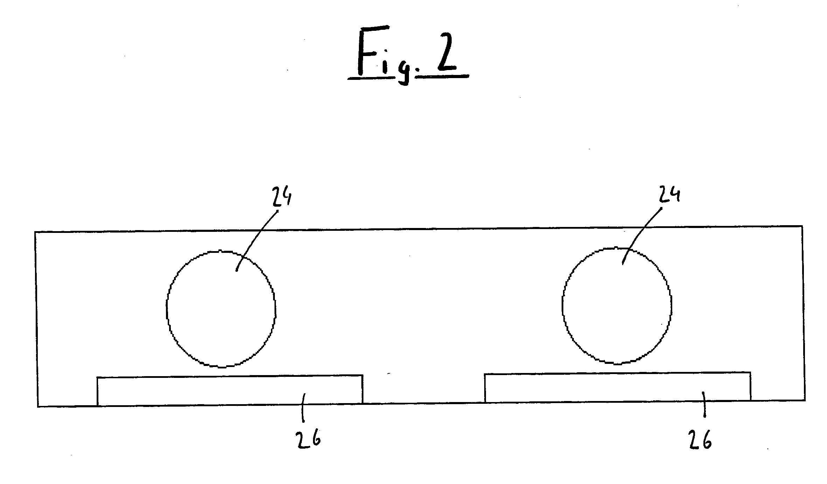

Der verbleibende Zwischenraum zwischen Objektträger 20 und Filz- oder Schaumstoffschicht ist auch für die Luftzirkulation notwendig, die in Fig. 1 und 3 veranschaulicht ist. Im gezeigten Ausführungsbeispiel sind zwei Lüfter 24 an der Rückseite der Inkubationskammer 10 vorgesehen, die die Luft durch Luftkanäle 26 (siehe Fig. 2) aus dem Zwischenraum zwischen Objektträger 20 und Wasserreservoir 22 absaugen und in den Bereich zwischen der heizbaren Deckelplatte 14 und dem Objektträger 20 einblasen. Auf diese Weise wird der Verdunstungsvorgang begünstigt und ein gleichmäßiger Feuchtegehalt im gesamten Inneren 12 der Kammer sichergestellt.

Durch die mit Feuchtigkeit gesättigte Luft, die direkt an den Objektträgern bzw. den darauf angebrachten und mit Enzymen beschichteten Gewebeschnitten vorbeigeführt wird, erreicht man eine gleichbleibende mit Wasser gesättigte Atmosphäre, die für ein ausreichendes und reproduzierbares Verdauergebnis ausschlaggeben ist. Durch die heizbare Deckelplatte 14 wird sichergestellt, dass eine Kondensatbildung an der Unterseite des Deckels ausgeschlossen ist, so dass keine Gefahr besteht, dass Kondensattropfen auf die Objektträger 20 herabtropfen können, was den Verdauprozess beeinträchtigen und zumindest die Ortsinformationen an den Probeschnitten beeinträchtigen könnte. Ein Feuchtigkeitssensor 28 im Kammerinneren überwacht die Feuchtigkeit ständig, so dass diese im Bereich der Sättigung um 100% gehalten werden kann. Ein Temperatursensor (nicht gezeigt) dient dem Einstellen der gewünschten Temperatur, wobei die Inkubationskammer 10 ein Aufheizen bis 60°C ermöglicht, da es sich gezeigt hat, dass die Verdauprozesse bei einigen Enzymen bei Reaktionstemperaturen über 37°C erheblich schneller ablaufen.

Die Kühlfunktion hat den Vorteil, dass die Objektträger während des Auftragens der Enzyme in mehreren Schichten unter der Umgebungstemperatur gehalten werden können, so dass die Enzyme besser in das Gewebe eindringen können, bevor der Verdauprozess dann zu einem definierten Zeitpunkt gestartet wird.

Die Inkubationskammer 10 ist durch eine geeignete Dichtung verschlossen, um unnötige Verluste an Feuchtigkeit einerseits und eine sicheren Abschottung von der außerhalb der Inkubationskammer 10 liegenden Atmosphäre andererseits sicherzustellen.

Die Temperatur kann innerhalb der Inkubationskammer auch an mehreren Stellen gemessen und durch die beiden Klimazonen auch getrennt geregelt werden. Der gesamte Verlauf eines Verdauprozesses kann dabei von der Steuerelektronik protokolliert und später ausgedruckt werden, so dass der Anwender wertvolle Informationen erhält, die hilfreich sein können, die Verdauparameter für bestimmte Anwendungen zu optimieren bzw. die allseits gewünschte Reproduzierbarkeit des Verdauprozesses zu ermöglichen. Die Temperatur der heizbaren Deckelplatte 14 kann aktiv gesteuert oberhalb der Temperatur der beheizten Bodenplatte liegen, um Kondensationsvorgänge an der Unterseite sicher zu vermeiden.

## Patentansprüche

1. Inkubationskammer mit einem Feuchtigkeitsreservoir (22), einer Klimatisierungseinrichtung, die die Einstellung einer gewünschten Temperatur ermöglicht, sowie einem Objekthalter (18) für biologische oder chemische Proben, **dadurch gekennzeichnet, dass** die Klimatisierungseinrichtung über zwei getrennte Klimatisierungszonen (14, 16) verfügt, die getrennt voneinander regelbar sind, wobei eine erste Klimatisierungszone (14) im Bereich des Deckels der Kammer angeordnet ist und Temperatur- und Feuchtigkeitssensoren (28) in der Kammer (12) vorgesehen sind, die die Temperatur und die Luftfeuchtigkeit in der Kammer (12) in Abhängigkeit von den gemessenen Werten über eine Steuervorrichtung steuern.

2. Inkubationskammer nach Anspruch 1, **dadurch gekennzeichnet, dass** wenigstens eine der beiden Klimatisierungszonen (16) auch eine Kühlfunktion aufweist.

3. Inkubationskammer nach Anspruch 1 oder 2, **dadurch gekennzeichnet, dass** die zweite Klimatisierungszone (16) **in** der Bodenplatte der Inkubationskammer angeordnet ist.

4. Inkubationskammer nach einem der Ansprüche 1 bis 3,
**dadurch gekennzeichnet, dass** das Feuchtigkeitsreservoir (22) durch eine Wanne ausgebildet ist, deren aktive Oberfläche durch eine Filz- oder Schaumstofflage vergrößert ist.

5. Inkubationskammer nach Anspruch 4, **dadurch gekennzeichnet, dass** der Objekthalter (18) und die Wanne (22) aus wärmeleitendem Metall und/oder Kunststoff bestehen.

6. Inkubationskammer nach einem der vorhergehenden Ansprüche, **dadurch gekennzeichnet, dass** die Klimatisierungseinrichtung (14, 16) derart ausgelegt ist, dass eine Temperatur in der Kammer zwischen 4°C und 60°C einstellbar ist.

7. Inkubationskammer nach einem der vorhergehenden Ansprüche, **dadurch gekennzeichnet, dass** der Objekthalter (18) für die Aufnahme von Objektträgern (20) und/oder MALDI-Probenträgern ausgebildet ist, wobei die Objekt- und/oder Probenträger in einem bestimmten Abstand zum Feuchtigkeitsreservoir (22) gehalten sind.

8. Inkubationskammer nach Anspruch 7, **dadurch gekennzeichnet, dass** die Proben auf den Objekt- oder Probenträgern wahlweise nach unten zum Feuchtigkeitsreservoir oder nach oben zum freien Raum hin anordenbar sind.

9. Inkubationskammer nach einem der vorhergehenden Ansprüche, **dadurch gekennzeichnet, dass** eine Lüftervorrichtung (24) vorgesehen ist, mit der die Luft innerhalb der Inkubationskammer an den Objekt- oder Probenträgern entlang in Zirkulation versetzbar ist.

10. Inkubationskammer nach Anspruch 9, **dadurch gekennzeichnet, dass** die Leistung der Lüftervorrichtung zwischen 0 und einem Maximalwert regelbar ist.

11. Inkubationskammer nach einem der vorhergehenden Ansprüche, **dadurch gekennzeichnet, dass** der Deckel (14) der Kammer (10) abnehmbar oder aufschwenkbar ist.

12. Inkubationskammer nach einem der vorhergehenden Ansprüche, **dadurch gekennzeichnet, dass** der Innenraum (12) der Kammer durch eine geschlossen umlaufende Dichtung zur Umgebung abgedichtet ist.

## Claims

1. An incubation chamber with a moisture reservoir (22), a climate conditioner which enables a desired temperature to be set, and an object holder (18) for biological or chemical specimens, **characterized in that** the climate conditioner has two separate conditioning zones (14, 16) which are regulatable separately from one another, whereby the first conditioning zone (14) is arranged in the vicinity of the cover of the chamber and temperature and humidity sensors (28) are provided in the chamber (12), which control the temperature and the humidity in the chamber (12) via a control device depending on the measured values.

2. The incubation chamber of claim 1, **characterized in that** at least one of the two conditioning zones (16) also has a cooling function.

3. The incubation chamber of claim 1 or 2, **characterized in that** the second conditioning zone (16) is located in the base plate of the incubation chamber.

4. The incubation chamber of one of claims 1 to 3, **characterized in that** the moisture reservoir (22) is embodied by a tub, an active surface area of which is increased by a layer of felt or foam.

5. The incubation chamber of claim 4, **characterized in that** the object holder (18) and the tub (22) consist of heat-conducting metal and/or plastic.

6. The incubation chamber of one of the previous claims, **characterized in that** the climate conditioner (14, 16) is designed such that a temperature in the chamber of between 4° C. and 60° C. can be set.

7. The incubation chamber of one of the previous claims, **characterized in that** the object holder (18) is embodied for receiving object carriers (20) and/or MALDI sample carriers, and the object carriers and/or sample carriers are held at a defined spacing from the moisture reservoir (22).

8. The incubation chamber of claim 7, **characterized in that** the samples on the object carriers or sample carriers can be located selectively downward toward the moisture reservoir or upward toward the free space.

9. The incubation chamber of one of the previous claims, **characterized in that** a fan apparatus (24) is provided, with which the air inside the incubation chamber can be put into circulation along the object carriers or sample carriers.

10. The incubation chamber of claim 9, **characterized in that** the power of the fan apparatus can be regulated between 0 and a maximum value.

11. The incubation chamber of one of the previous claims, **characterized in that** the cover (14) of the chamber is removable or pivotable.

12. The incubation chamber of claim 1, **characterized in that** the interior (12) of the chamber is sealed off from the surroundings by an unbroken surrounding seal.

## Revendications

1. Chambre d'incubation avec un réservoir d'humidité (22), avec un dispositif de climatisation qui permet le réglage d'une température souhaitée ainsi qu'avec un support d'objet (18) pour des échantillons biologiques ou chimiques, **caractérisée en ce que** le dispositif de climatisation dispose de deux zones de climatisation séparées (14, 16) qui peuvent être régulées séparément l'une de l'autre, une première zone de climatisation (14) étant agencée dans la zone du couvercle de la chambre et des capteurs de température et d'humidité (28) étant prévus dans la chambre (12), lesquels commandent par l'intermédiaire d'un dispositif de commande la température et l'humidité de l'air dans la chambre (12) en fonction des valeurs mesurées.

2. Chambre d'incubation selon la revendication 1, **caractérisée en ce qu'**au moins l'une des deux zones de climatisation (16) a aussi une fonction de refroidissement.

3. Chambre d'incubation selon la revendication 1 ou 2, **caractérisée en ce que** la deuxième zone de climatisation (16) est agencée dans la plaque de fond de la chambre d'incubation.

4. Chambre d'incubation selon l'une des revendications 1 à 3, **caractérisée en ce que** le réservoir d'humidité (22) est constitué d'une cuve dont la surface active est augmentée par une couche de feutre ou de mousse.

5. Chambre d'incubation selon la revendication 4, **caractérisée en ce que** le support d'objet (18) et la cuve (22) sont en plastique et/ou métal thermoconducteur.

6. Chambre d'incubation selon l'une des revendications précédentes, **caractérisée en ce que** le dispositif de climatisation (14, 16) est conçu de telle sorte qu'une température dans la chambre est réglable entre 4°C et 60°C.

7. Chambre d'incubation selon l'une des revendications précédentes, **caractérisée en ce que** le support d'objet (18) est conçu pour recevoir des porte-objets (20) et/ou des porte-échantillons MALDI, les porte-objets et/ou porte-échantillons étant maintenus à une certaine distance du réservoir d'humidité (22).

8. Chambre d'incubation selon la revendication 7, **caractérisée en ce que** les échantillons peuvent être agencés sur les porte-objets ou porte-échantillons, au choix vers le bas en direction du réservoir d'humidité ou vers le haut en direction de l'espace libre.

9. Chambre d'incubation selon l'une des revendications précédentes, **caractérisée en ce qu'**il est prévu un dispositif de ventilation (24) avec lequel l'air à l'intérieur de la chambre d'incubation peut être mis en circulation le long des porteobjets et/ou porte-échantillons.

10. Chambre d'incubation selon la revendication 9, **caractérisée en ce que** la puissance du dispositif de ventilation est réglable entre 0 et une valeur maximale.

11. Chambre d'incubation selon l'une des revendications précédentes, **caractérisée en ce que** le couvercle (14) de la chambre (10) peut être enlevé ou soulevé par pivotement.

12. Chambre d'incubation selon l'une des revendications précédentes, **caractérisée en ce que** l'espace intérieur (12) de la chambre est rendu étanche par rapport à l'environnement par un joint d'étanchéité qui en fait le tour complet.
